(19) Europäisches Patentamt
European Patent Office
Office européen des brevets

(11) EP 3 004 857 B1

(12) **EUROPEAN PATENT SPECIFICATION**

(45) Date of publication and mention
of the grant of the patent:
**29.08.2018 Bulletin 2018/35**

(51) Int Cl.:
***G01N 27/327*** (2006.01)    ***C12Q 1/00*** (2006.01)

(21) Application number: **14732114.5**

(86) International application number:
**PCT/EP2014/061881**

(22) Date of filing: **06.06.2014**

(87) International publication number:
**WO 2014/195480 (11.12.2014 Gazette 2014/50)**

(54) **ELECTROCHEMICAL-BASED ANALYTICAL TEST STRIP WITH A SOLUBLE ELECTROCHEMICALLY-ACTIVE COATING OPPOSITE A BARE ELECTRODE**

ANALYTISCHER TESTSTREIFEN AUF ELEKTROCHEMISCHER BASIS MIT EINER LÖSLICHEN ELEKTROCHEMISCH AKTIVEN BESCHICHTUNG GEGENÜBER EINER BLANKEN ELEKTRODE

BANDE DE TEST ANALYTIQUE À BASE ÉLECTROCHIMIQUE AVEC UN REVÊTEMENT ÉLECTROCHIMIQUEMENT ACTIF SOLUBLE OPPOSÉ À UNE ÉLECTRODE NUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.06.2013 GB 201310211**

(43) Date of publication of application:
**13.04.2016 Bulletin 2016/15**

(73) Proprietor: **Lifescan Scotland Limited
Inverness IV2 3ED (GB)**

(72) Inventors:
• **COOPER, Alexander
Inverness, Invernessshire IV2 3ED (GB)**

• **RODGERS, James Iain
Inverness, Invernessshire IV2 3ED (GB)**
• **MACFIE, Gavin
Inverness, Invernessshire IV2 3ED (GB)**

(74) Representative: **Brunner, John Michael Owen
Carpmaels & Ransford LLP
One Southampton Row
London WC1B 5HA (GB)**

(56) References cited:
**EP-A1- 0 225 061     EP-A1- 1 707 953
EP-A1- 1 742 045     EP-A2- 0 537 761**

**Description**

**FIELD OF THE INVENTION**

The present invention relates, in general, to medical devices and, in particular, to analytical test strips and related methods.

**BACKGROUND OF THE INVENTION**

The determination (e.g., detection and/or concentration measurement) of an analyte in, or a characteristic of, a fluid sample is of particular interest in the medical field. For example, it can be desirable to determine glucose, ketone bodies, cholesterol, lipoproteins, triglycerides, acetaminophen, hematocrit and/or HbA1c concentrations in a sample of a bodily fluid such as urine, blood, plasma or interstitial fluid. Such determinations can be achieved using analytical test strips, based on, for example, visual, photometric or electrochemical techniques. Conventional electrochemical-based analytical test strips are described in, for example, U.S. Patent Nos. 5,708,247, and 6,284,125, each of which is hereby incorporated in full by reference. EP0225061A1 describes a potentiometric electrochemical analysis method, which comprises placing an aqueous sample liquid possibly containing a quantity of an analyte which is to be the subject of the test, in each of two compartments of an electrochemical analysis cell, wherein the liquid in each compartment is placed in electrochemical contact with one of two electrodes forming part of the cell, wherein the electrodes are insensitive to the analyte, and wherein the compartments are separated by an electroactive barrier which is sensitive to or selective for the analyte, said barrier not being in electrical contact with either electrode except through the medium of the sample liquid, said method further comprising changing the amount of the analyte material in one of the compartments by a predefined standard quantity, and measuring the cell potential to give a result from which a measure of the concentration of analyte in the sample can be obtained by data-processing or calculation. EP1742045A1 describes a method of measuring a component in blood, by which the amounts of blood cells and an interfering substance can be measured with high accuracy and high reliability and the amount of the component can be corrected accurately based on the amounts of the blood cells and the interfering substance. In a sensor for measuring a blood component, a first working electrode 13 measures a current that flows during a redox reaction of a blood component, a second working electrode 17 measures the amount of blood cells, and a third working electrode 12 measures the amount of an interfering substance. Next, based on the measurement results, the amount of the blood component to be measured is corrected.

**SUMMARY OF THE INVENTION**

The invention is defined in the independent claims. There is provided an electrochemical-based analytical test strip for the determination of an analyte in a bodily fluid sample, the electrochemical-based analytical test strip comprising:

an electrically insulating base layer; a patterned electrically conductive layer disposed on the electrically insulating base layer and including a plurality of electrodes; an enzymatic reagent layer disposed on a portion of the patterned conductor layer to define at least one bare electrode and a plurality of enzymatic reagent covered electrodes from the plurality of electrodes; a patterned spacer layer; a top layer having an underside surface; and a soluble electrochemically-active coating on the underside surface of the top layer;
wherein at least the patterned spacer layer and top layer define a sample-receiving chamber within the electrochemical-based analytical test strip;
and wherein the soluble electrochemically-active coating is disposed on the underside surface of the top layer within at least a portion the sample-receiving chamber and in an opposing relationship to the bare electrodes.

The soluble electrochemically-active coating may include a redox agent.
The soluble electrochemically-active coating may include an enzymatic mediator.
The soluble electrochemically-active coating may include ferricyanide.
The soluble electrochemically-active coating is enzyme-free.
The at least one bare electrode may be one bare electrode.
The soluble electrochemically-active coating may be disposed opposite the at least one bare electrode and spaced apart from the plurality of enzymatic reagent covered electrodes.
The soluble electrochemically-active coating may be spaced apart from the plurality of enzymatic reagent covered electrodes by a distance in the range of 150 micrometers to 450 micrometers.
The at least one bare electrode may be configured to generate a current response upon the introduction of a bodily fluid sample into the sample-receiving chamber that is measurable by an associated test meter.
The bodily fluid sample may be a whole blood sample and the current response of the at least one bare electrode may be dependent on hematocrit of the whole blood sample.

An electrochemical response of the bare electrode may independent of analyte concentration of the bodily fluid sample

The bodily fluid sample may be a whole blood sample.

At least the top layer and soluble electrochemically-active coating may be integrated as an engineered top tape.

The analyte may be glucose and the bodily fluid sample may be a whole blood sample.

The soluble electrochemically-active coating and at least one bare electrode of the patterned electrically conductor layer may be separated by a vertical distance of in the range of approximately 50 micrometers to approximately 150 micrometers in the sample-receiving chamber.

In a second aspect of the invention, there is provided a method for employing an analytical test strip, the method comprising: introducing a blood sample into a sample-receiving chamber of an electrochemical-based analytical test strip, the electrochemical-based analytical test strip including: a top layer with an underside surface; at least one bare electrode in the sample-receiving chamber; and a soluble electrochemically-active coating on the underside surface within at least a portion the sample-receiving chamber and in an opposing relationship to the at least one bare electrode, and wherein the introduction is such that the soluble electrochemically-active coating operably dissolves in the bodily fluid sample; detecting an electrochemical response of the at least one bare electrode of the electrochemical-based analytical test strip; and determining an analyte in the blood sample based in part on the detected electrochemical response of the at least one bare electrode.

The electrochemical-based analytical test strip includes: an electrically insulating base layer; a patterned electrically conductive layer disposed on the electrically insulating base layer and including a plurality of electrodes; an enzymatic reagent layer disposed on at least a portion of the patterned electrically conductor layer to define the at least one bare electrode and a plurality of enzymatic reagent covered electrodes from the plurality of electrodes and a patterned spacer layer; and wherein at least the patterned spacer layer and top layer define the sample-receiving chamber within the electrochemical-based analytical test strip.

The detecting of an electrochemical response may also include detecting an electrochemical response of the plurality of enzymatic reagent covered electrodes.

The soluble electrochemically-active coating may contain an enzymatic mediator.

The soluble electrochemically-active coating may contain a redox agent.

The soluble electrochemically-active coating is enzyme-free.

The plurality of bare electrodes may be one bare electrode.

The soluble electrochemically-active coating may be spaced apart from the plurality of enzymatic reagent covered electrodes.

The electrochemical response of at least one bare electrode may be a current response.

The bodily fluid sample may be a whole blood sample and the current response of the at least one bare electrode may be dependent on hematocrit of the whole blood sample.

An electrochemical response of the at least one bare electrode may be independent of analyte concentration of the bodily fluid sample.

The blood sample may be a whole blood sample.

The analyte may be glucose and the blood sample may be a whole blood sample.

The soluble electrochemically-active coating and the at least one bare electrodes of the patterned electrically conductor layer may be separated by a vertical distance in the range of approximately 50 micrometers to approximately 150 micrometers in the sample-receiving chamber.

## BRIEF DESCRIPTION OF THE DRAWINGS

The accompanying drawings, which are incorporated herein and constitute part of this specification, illustrate presently preferred embodiments of the invention, and, together with the general description given above and the detailed description given below, serve to explain features of the invention, in which:

FIG. 1 is a simplified exploded perspective view of an electrochemical-based analytical test strip according to an embodiment of the present invention;

FIG. 2 is a simplified perspective view of the electrochemical-based analytical test strip of FIG. 1;

FIG. 3 is a simplified cross-sectional side view of a portion of the electrochemical-based analytical test strip of FIG. 1 taken along line A-A of FIG. 2;

FIG. 4 is a graphical depiction of electrochemical response current transients from a bare electrode of an electrochemical-based analytical test strip according to the present invention;

FIG. 5 is a graphical depiction of glucose determination measurement bias for a conventional electrochemical-based analytical test strip and an electrochemical-based analytical test strip according to the present invention; and

FIG. 6 is a flow diagram depicting stages in a method for determining an analyte in a bodily fluid sample according to an embodiment of the present invention.

## DETAILED DESCRIPTION OF ILLUSTRATIVE EMBODIMENTS

The following detailed description should be read with reference to the drawings, in which like elements in different drawings are identically numbered. The drawings, which are not necessarily to scale, depict exemplary embodiments for the purpose of explanation only and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein.

In general, electrochemical-based analytical test strips for the determination of an analyte (such as glucose) in a bodily fluid sample (for example, a whole blood sample) according to embodiments of the present invention includes an electrically insulating base layer, a patterned electrically conductive layer disposed on the electrically insulating base layer that includes a plurality of electrodes, and an enzymatic reagent layer disposed on a portion of the patterned conductor layer and defining at least one bare electrode and a plurality of enzymatic reagent covered electrodes from the plurality of electrodes. Electrochemical-based analytical test strips according to the present invention also include a patterned spacer layer, a top layer having an underside surface, and a soluble electrochemically-active coating (for example, a soluble redox agent containing coating or a soluble mediator-containing coating such as a ferrocyanide-containing coating) disposed on the underside surface of the top layer. In addition, at least the patterned spacer layer and top layer define a sample-receiving chamber within the electrochemical-based analytical test strip. Moreover, the soluble electrochemically-active coating is disposed on the underside surface of the top layer within at least a portion the sample-receiving chamber and in an opposing relationship to the bare electrodes.

The determination of an analyte in a bodily fluid sample, such as the determination of glucose in a whole blood sample, using electrochemical-based analytical test strips can be susceptible to determination inaccuracies arising from variation in characteristics of the whole blood sample. For example, the hematocrit of whole blood samples is known to affect the accuracy of glucose determination in whole blood samples.

Electrochemical-based analytical test strips according to embodiments of the present invention are beneficial in that, for example, the soluble electrochemically-active coating results in an electrochemical response (such as a current response) at the bare electrode that can be used to compensate (i.e., correct) electrochemical responses from the enzymatic reagent covered electrodes for hematocrit affects or other bodily fluid samples that affect the diffusion rate of the soluble electrochemically-active component of the coating such as temperature and viscosity. It is hypothesized, without being bound, that an electrochemically-active component (such as a redox agent or mediator) from the soluble electrochemically-active coating diffuses through a bodily fluid sample at a rate that is dependent on hematocrit and that, therefore, the timing and/or magnitude of the electrochemical response at the bare electrode is an indirect measurement of the hematocrit and, therefore, can be employed to beneficially improve the accuracy of analyte determination.

Electrochemical-based analytical test strips according to the present invention are also beneficial in that they only require a single bare electrode, thus enabling a sample-receiving chamber that is advantageously low in volume. However, if desired to provide redundancy or to improve accuracy, a plurality of bare electrodes can be employed in embodiments of the present invention. In addition, the electrochemical response of the bare electrode can be, for example, a current transient that is simply and inexpensively measured using typical test meter current measurement circuitry.

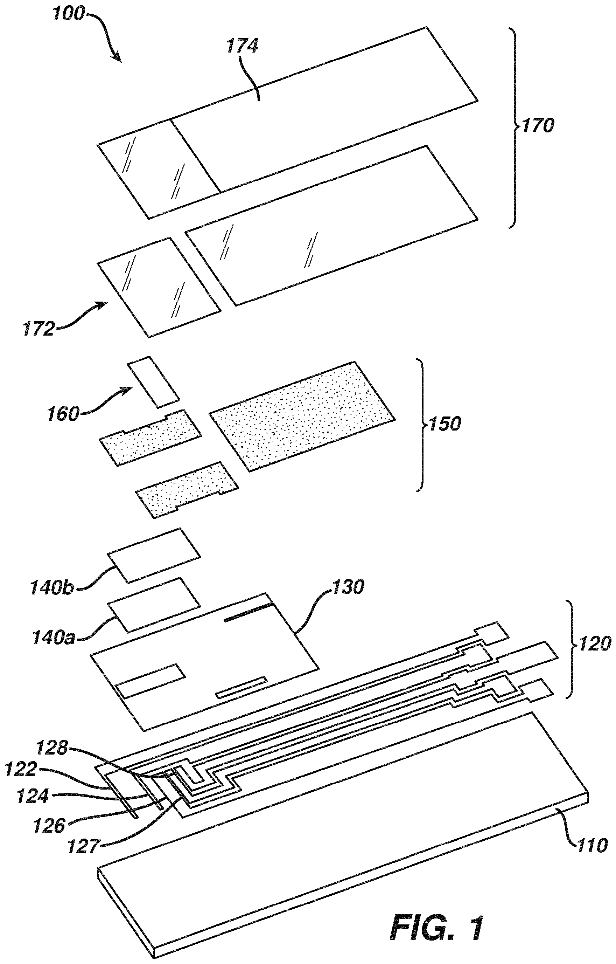

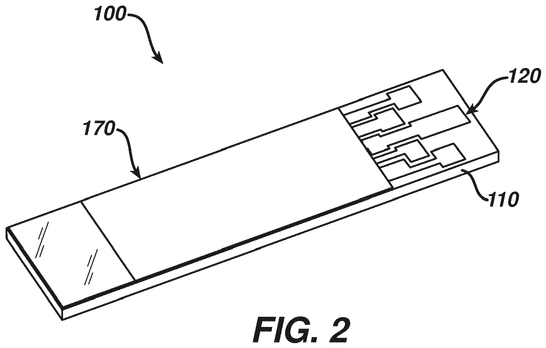

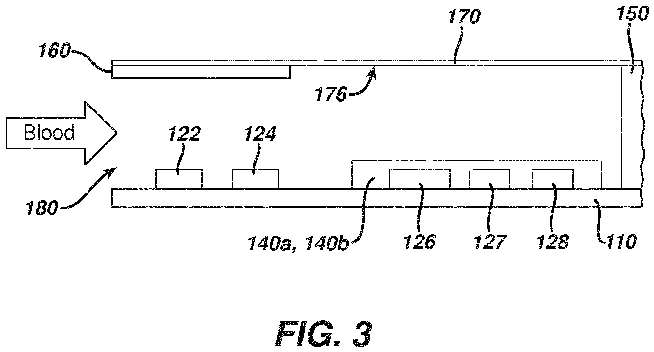

FIG. 1 is a simplified exploded perspective view of an electrochemical-based analytical test strip 100 according to an embodiment of the present invention. FIG. 2 is a simplified perspective view of electrochemical-based analytical test strip 100. FIG. 3 is a simplified cross-sectional side view of a portion of electrochemical-based analytical test strip 100 taken along line A-A of FIG. 2.

Referring to FIGs. 1-3, electrochemical-based analytical test strip 100 for the determination of an analyte (such as glucose) in a bodily fluid sample (for example, a whole blood sample) includes an electrically-insulating base layer 110, a patterned electrically conductive layer 120, an optional patterned insulation layer 130, enzymatic reagent layers 140a and 140b, a patterned spacer layer 150, a soluble electrochemically-active coating 160, a top layer 170 consisting of a patterned hydrophilic sub-layer 172 and a top tape 174. Patterned hydrophilic sub-layer 172 of top layer 170 has an underside surface 176 (see FIG. 3 in particular).

Patterned electrically conductive layer 120 is disposed on electrically-insulating base layer 110 and includes a plurality of electrodes (i.e., bare electrodes 122 and 124 and enzymatic reagent covered electrodes 126, 127 and 128 as described further herein). Enzymatic reagent layers 140a and 140b are disposed on a portion of the patterned electrically conductor layer 120 to define bare electrodes 122 and 124 and enzymatic reagent covered electrodes 126, 127 and 128 (see FIG. 3 in particular wherein enzymatic reagent layers 140a and 14b are depicted as a single layer for simplicity). Soluble electrochemically-active coating 160 is disposed on underside surface 176 of top layer 170 within at least a portion of a sample-receiving chamber 180 and in an opposing relationship to bare electrodes 122 and 124 (see FIG. 3 in particular).

In the embodiment of FIGs. 1-3, at least the patterned spacer layer and top layer define a sample-receiving chamber 180 within electrochemical-based analytical test strip 100 (see FIG. 3 in particular where the introduction of a bodily fluid sample (i.e., blood) into sample-receiving chamber 180 is depicted with an arrow).

Electrically-insulating base layer 110 can be any suitable electrically-insulating base layer known to one skilled in the art including, for example, a nylon base layer, a polycarbonate base layer, a polyimide base layer, a polyvinyl chloride base layer, a polyethylene base layer, a polypropylene base layer, a glycolated polyester (PETG) base layer, or a polyester base layer. The electrically-insulating base layer can have any suitable dimensions including, for example, a width dimension of about 5 mm, a length dimension of about 27 mm and a thickness dimension of about 0.5 mm.

Electrically-insulating base layer 110 provides structure to electrochemical-based analytical test strip 100 for ease of handling and also serves as a base for the application (e.g., printing or deposition) of subsequent layers (e.g., a patterned electrically conductor layer).

Enzymatic reagent covered electrodes 126, 127, and 128 can be, for example, configured such that enzymatic reagent electrode 126 serves as a counter/reference electrode, enzymatic reagent covered electrode 127 serves as a first working electrode and enzymatic reagent covered electrode 128 serves as a second working electrode, respectively. Although, for the purpose of explanation only, electrochemical-based analytical test strip 100 is depicted as including a total of three enzymatic reagent covered electrodes, embodiments of electrochemical-based analytical test strips, including embodiments of the present invention, can include any suitable number of such electrodes.

Bare electrodes 122 and 124 and enzymatic reagent covered electrodes 126, 127 and 128 of electrochemical-based analytical test strip 100 can be formed of any suitable conductive material including, for example, gold, palladium, platinum, indium, titanium-palladium alloys and electrically conducting carbon-based materials including carbon inks. It should be noted that patterned electrically conductive layers employed in analytical test strips according to embodiments of the present invention can take any suitable shape and be formed of any suitable materials including, for example, metal materials and conductive carbon materials.

Referring in particular to FIGs. 1 and 3, the configuration and disposition of enzymatic reagent covered electrodes 126, 127 and 128 and enzymatic reagent layers 140a and 140b are such that electrochemical-based analytical test strip 100 is configured for the electrochemical determination of an analyte (such as glucose) in a bodily fluid sample (such as, for example, a whole blood sample with a physiological hematocrit content) that has filled sample-receiving chamber 180.

Enzymatic reagent layer 140 is disposed on only a portion of patterned electrically conductive layer 120 such that bare electrodes 122 and 124 are defined (see FIG. 3). Only a single (i.e., one) bare electrode is required in electrochemical-based analytical test strips according to embodiments of the present invention. However, the embodiment of FIGs. 1-3 includes two bare electrodes for redundancy.

Enzymatic reagent layers 140a and 140b can include any suitable enzymatic reagents, with the selection of enzymatic reagents being dependent on the analyte to be determined. For example, if glucose is to be determined in a blood sample, enzymatic reagent layers 140a and 140b can include a glucose oxidase or glucose dehydrogenase along with other components necessary for functional operation. Enzymatic reagent layer 140a and 140b can include, for example, glucose oxidase, tri-sodium citrate, citric acid, polyvinyl alcohol, hydroxyl ethyl cellulose, potassium ferricyanide, potassium ferrocyanide, antifoam, fumed silica (either with or without a hydrophobic surface modification), PVPVA, and water. Further details regarding reagent layers, and electrochemical-based analytical test strips in general, are in U.S. Patent Nos. 6,241,862 and 6,733,655. It should be noted that the amount of acidic material employed in enzymatic reagents (such as the citric acid and tri-sodium citrate mentioned above) is not sufficient to reduce the pH of a bodily fluid sample to the levels required to provide beneficially reduced interferent effects.

Patterned spacer layer 150 can be formed, for example, from a screen-printable pressure sensitive adhesive commercially available from Apollo Adhesives, Tamworth, Staffordshire, UK. In the embodiment of FIGs. 1 through 3, patterned spacer layer 150 defines outer walls of the sample-receiving chamber 180. Patterned spacer layer 150 can have a thickness of, for example, approximately 75 microns, be electrically nonconductive, and be formed of a polyester material with top and bottom side acrylic-based pressure sensitive adhesive.

Soluble electrochemically-active coating 160 is disposed on the underside surface 176 of patterned hydrophilic sub-layer 172 of top layer 170 within at least a portion of sample-receiving chamber 180 such that soluble electrochemically-active coating 160 is disposed above bare electrodes 122 and 124. Moreover, soluble electrochemically-active coating 160 is operably dissolvable in the bodily fluid sample such that, during use of electrochemical-based analytical test strip 100, an electrochemically-active component (such as a redox agent or mediator) of the soluble electrochemically-active coating will diffuse from the vicinity of underside surface 176 to bare electrodes 122 and 124 giving rise to an electrochemical response at bare electrodes 122 and 124. Since the diffusion rate of the electrochemically-active component is dependent on, for example, hematocrit of the bodily fluid sample, the timing and magnitude of the electrochemical response will depend on the hematocrit of the bodily fluid sample. The electrochemical response can be used in a suitable algorithm, to determine hematocrit and/or to compensate for the effect of hematocrit on glucose determination based on an electrochemical response of enzymatic reagent covered electrodes 126, 127 and 128.

It should be noted that in the embodiment of FIGs. 1-3, the soluble electrochemically-active coating is disposed in an opposing relationship to (i.e., opposite) bare electrodes 122 and 124, but is not in an opposing relationship to enzymatic reagent covered electrodes 126, 127 and 128. Therefore, soluble electrochemically-active coating 160 does not significantly affect the electrochemical response of enzymatic reagent covered electrodes 126, 127 and 128. In addition, the soluble electrochemically-active coating is enzyme free, therefore eliminating any analyte concentration dependent enzymatic reagent effect on the electrochemical response of the bare electrode(s).

Soluble electrochemically-active coating 160 can contain any suitable electrochemically-active component that can undergo a redox reaction at the working potential of the bare electrode(s) including, for example, the reduced or oxidized forms of enzymatic mediators such as Potassium Ferricyanide, water soluble ferrocenes, water soluble ferrocinium salts, osmium complexes, quinones (such as hydroquinone, benzoquinone, phenanthrocquinones and derivatives thereof) phenathiozine derivatives, and Meldola's blue. Moroever, the electrochemically-active component can be a redox agent capable of undergoing at least electrode oxidation or electrode reduction. Such redox agents include, for example, iodide, iodine, thiosulfate salts, soluble oxidisable metal salts such as nitrates, chlorides, sulfates and phosphates of silver and copper along with other transition metals, complexes of manganese (e.g. potassium permanganate) or chromium (e.g. potassium dichromate), hypochlorite salts, ammonium and organic amine salts, and unsaturated organic compounds such as maleic anhydride. Soluble electrochemically-active coating 160 is typically enzyme-free to avoid creating an analyte-dependent response at the bare electrodes.

Soluble electrochemically-active coating 160 and the at least one bare electrodes of the patterned electrically conductor layer are separated by a vertical distance in the range of, for example, approximately 50 microns to approximately 150 microns in the sample-receiving chamber. If desired to avoid compensating for background response, the soluble electrochemically-active coating can be spaced apart from the plurality of enzymatic reagent covered electrodes by a distance in the range of, for example, 150 microns to 450 microns.

Top layer 170 can be, for example, a clear film with hydrophilic properties that promote wetting and filling of electrochemical-based analytical test strip 100 by a fluid sample (e.g., a whole blood sample). Such clear films are commercially available from, for example, 3M of Minneapolis, Minnesota U.S.A. and Coveme (San Lazzaro di Savena, Italy). Top layer 170 can be, for example, a polyester film coated with a surfactant that provides a hydrophilic contact angle < 10 degrees. Top layer 170 can also be a polypropylene film coated with a surfactant or other surface treatment. In such a circumstance, the surfactant coating serves as patterned hydrophilic sub-layer 172. Moreover, if desired, the soluble acidic material coating can be formulated as a hydrophilic coating and also serve as a patterned hydrophilic sub-layer. Top layer 170 can have a thickness, for example, of approximately $100\mu m$.

Electrochemical-based analytical test strip 100 can be manufactured, for example, by the sequential aligned formation of patterned electrically conductive layer 120, patterned insulation layer 130, enzymatic reagent layers 140a and 140b, patterned spacer layer 150, mediator containing layer 160 and patterned hydrophilic sub-layer 172 onto electrically-insulating base layer 110. Any suitable techniques known to one skilled in the art can be used to accomplish such sequential aligned formation, including, for example, screen printing, photolithography, photogravure, chemical vapour deposition and tape lamination techniques.

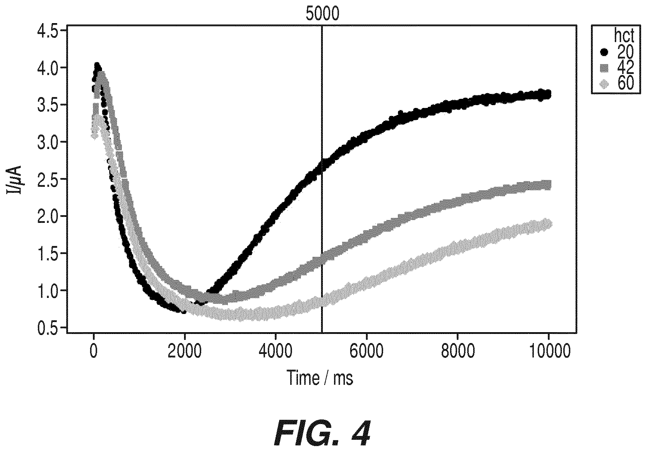

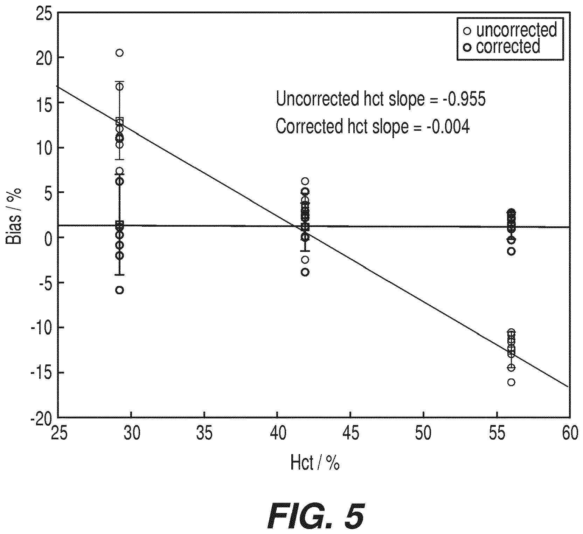

FIG. 4 is a graphical depiction of electrochemical response current transients from a bare electrode of an electrochemical-based analytical test strip according to the present invention. FIG. 4 depicts electrochemical response current transients for whole blood sample of three different hematocrit concentrations. FIG. 5 is a graphical depiction of glucose determination measurement bias for a conventional electrochemical-based analytical test strip and an electrochemical-based analytical test strip according to the present invention. In FIG. 5, the bias for the conventional electrochemical-based analytical test strip is labeled as "uncorrected" and the bias for the electrochemical-based analytical test strip according to the present invention is labeled "corrected." The label "corrected" refers to correction of the glucose determination based on the electrochemical response of the bare electrode following introduction of a whole blood sample into the sample-receiving chamber as described herein.

Prototype versions of electrochemical-based analytical test strip according to an embodiment of the present invention and as depicted in FIGs. 1-3 were created as follows. Ferrocyanide (obtained commercially from Sigma-Aldrich) was added to a 0.1M phosphate buffer (including dibasic and monobasic potassium phosphate obtained commercially from Sigma-Aldrich) to create a neutral pH 250mM ferrocyanide solution. Hydroxyethyl cellulose (HEC, commercially available from Ashlnd UK ltd., United Kingdom)), a thickening agent, was then added in the capacity of 0.1g per 10ml of solution to increase the viscosity of the ferrocyanide solution. The ferrocyanide solution was then applied to a top layer (layer 170 in FIGs. 1-3) such that it would be in an opposing relationship to bare electrodes 122 and 124 only and allowed to dry. The ferrocyanide containing coating thus prepared was approximately 2 microns in thickness. The top layer with ferrocyanide-containing coating was then used to manufacture electrochemical-based analytical test strips according to the present invention using standard web-based manufacturing techniques.

Prototypes were employed to collect electrochemical responses from the bare electrodes and the enzymatic reagent covered electrodes following introduction of whole blood samples into the prototypes and the application of a 400 mV bias to the prototypes. The whole blood samples had hematocrit levels of 20%, 42% and 60%, which is representative of physiological hematocrit levels. FIG. 4 depicts the electrochemical response transients for the bare electrodes.

The data of FIG. 4 demonstrate a clear difference in electrochemical current response between the three Hct levels at a measurement time of 5 seconds with the lowest Hct sample producing the greatest current response and the highest Hct sample producing the smallest current response. This indicates that the diffusion of ferrocyanide from the ferrocyanide-containing coating (i.e., the soluble electrochemically-active coating) to the bare electrode's surface can be used as a measure of Hct. Furthermore, the glucose response (i.e., the electrochemical response of the enzymatic reagent covered electrodes, not shown in FIG. 4) was not impacted by the ferrocyanide containing coating.

The electrochemical response data from the bare electrodes and the enzymatic reagent covered electrodes glucose was used to calculate a corrected current value that was then used to remove the Hct effect from a glucose determination as follows. First, a correction factor was calculated using the following algorithm:

$$I_{corr} = I_{WE} - C_1(I_{hct} - C_2)$$

where:

$I_{corr}$ = corrected current used to calculate measured glucose;

$I_{WE}$ = summed current measured at the two enzymatic reagent covered working electrodes at five seconds (which is dependent on both Hct and glucose)

$I_{hct}$ = current measured at a bare electrode at five seconds (which is Hct dependent but analyte (glucose) independent)

$C_1$ = 0.45, an experimentally determined coefficient; and

$C_2$ = 1.5, another experimentally determined coefficient.

Coefficients $C_1$ and $C_2$ were determined in the electrochemical response region of greatest hematocrit sensitivity. In practice and if desired, the algorithm could also include thresholds based on $I_{WE}$ such that when $I_{WE}$ is below a predetermined critical value a correction would not be applied. Where $I_{WE}$ is above a predetermined threshold value, a less aggressive correction using alternative values of $C_1$ and $C_2$ could be applied.

The determined (measured) glucose concentration was then calculated from the corrected current using the slope and intercept of an $I_{WE}$ versus glucose concentration relationship obtained by testing glucose spiked blood at a nominal Hct as follows:

$$\text{Measured glucose} = (I_{corr} - \text{glucose intercept})/(\text{glucose slope})$$

The corrected glucose value was then compared to a glucose measurement determined by a laboratory YSI instrument and bias between the two values calculated. This bias is plotted in FIG. 5 for both electrochemical-based analytical test strips according to the present invention and conventional electrochemical-based analytical test strips. The data of FIG. 5 indicates that use of electrochemical-based analytical test strips according to the present invention and the above described algorithms provides a beneficially flat slope of bias versus Hct (i.e., impact of Hct has been compensated) while conventional electrochemical-based analytical test strips in the absence of any correct algorithm exhibit a significant slope.

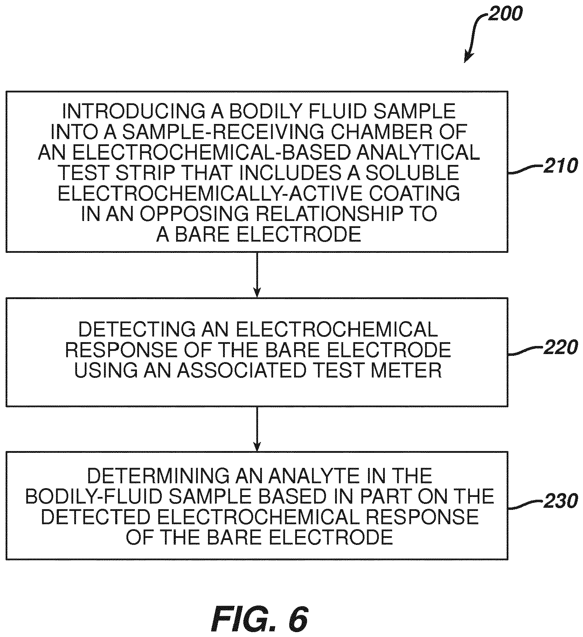

FIG. 6 is a flow diagram depicting stages in a method 200 for determining an analyte (such as glucose) in a bodily fluid sample (for example, a whole blood sample containing a physiological level of hematocrit) according to an embodiment of the present invention. Method 200 includes, at step 210, introducing a bodily fluid sample into a sample-receiving chamber of an electrochemical-based analytical test strip, the electrochemical-based analytical test strip including a top layer with an underside surface, at least one bare electrode in the sample-receiving chamber, and a soluble electrochemically-active coating on the underside surface. At least a portion of the soluble electrochemically-active coating is within the sample-receiving chamber and disposed in an opposing relationship to (I.e., opposite to) the at least one bare electrode. Moreover, the introduction of the bodily fluid sample is such that the soluble electrochemically-active coating operably dissolves in the bodily fluid sample.

At step 220 of method 200, an electrochemical response (such as a transient current response) of the at least one bare electrode of the electrochemical-based analytical test strip is detected using an associated test meter. An analyte in the bodily fluid sample is then determined based in part on the detected electrochemical response of the at least one bare electrode (see step 230 of FIG. 6).

Once apprised of the present disclosure, one skilled in the art will recognize that method 200 can be readily modified to incorporate any of the techniques, benefits, features and characteristics of electrochemical-based analytical test strips according to embodiments of the present invention and described herein.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that devices and methods within the scope of these claims and their equivalents be covered thereby.

**Claims**

1.  An electrochemical-based analytical test strip for the determination of an analyte in a blood sample, the electrochemical-based analytical test strip comprising:

    an electrically insulating base layer (110);
    a patterned electrically conductive layer (120) disposed on the electrically insulating base layer and including a plurality of electrodes;
    an enzymatic reagent layer (140a, 140b) disposed on a portion of the patterned conductive layer to define at least one bare electrode (122, 124) and a plurality of enzymatic reagent covered electrodes (126, 127, 128) from the plurality of electrodes;
    a patterned spacer layer (150);
    a top layer (170) having an underside surface (176); and
    a soluble electrochemically-active coating (160) on the underside surface of the top layer;

    wherein at least the patterned spacer layer and top layer define a sample-receiving chamber (180) within the electrochemical-based analytical test strip;
    wherein the soluble electrochemically-active coating is disposed on the underside surface of the top layer within at least a portion of the sample-receiving chamber and in an opposing relationship to the at least one bare electrode and not in an opposing relationship to the plurality of reagent covered electrodes; and
    wherein the soluble electrochemically-active coating is enzyme-free.

2.  The electrochemical-based analytical test strip of claim 1, wherein the soluble electrochemically-active coating is disposed opposite the at least one bare electrode and spaced apart from the plurality of enzymatic reagent covered electrodes, preferably spaced apart by a distance in the range of 150 micrometers to 450 micrometers.

3.  The electrochemical-based analytical test strip of claim 1 or claim 2, wherein the at least one bare electrode is configured to generate a current response upon the introduction of a blood sample into the sample-receiving chamber that is measurable by an associated test meter.

4.  The electrochemical-based analytical test strip of any one of claims 1 to 3, wherein at least the top layer and soluble electrochemically-active coating are integrated as an engineered top tape.

5.  The test strip of any one of claims 1 to 4, wherein the soluble electrochemically-active coating contains at least one of an enzymatic mediator and a redox agent.

6.  The test strip of any one of claims 1 to 4, wherein the soluble electrochemically-active coating and the at least one bare electrodes of the patterned electrically conductive layer are separated by a vertical distance in the range of 50 micrometers to 150 micrometers in the sample-receiving chamber.

7.  A method for employing an analytical test strip, the method comprising:
    introducing a blood sample into the sample-receiving chamber of the electrochemical-based analytical test strip of any one of claims 1 to 6;

wherein the introduction is such that the soluble electrochemically-active coating operably dissolves in the blood sample;

detecting an electrochemical response of the at least one bare electrode of the electrochemical-based analytical test strip; and

determining an analyte in the blood sample based in part on the detected electrochemical response of the at least one bare electrode.

8. The method of claim 7, wherein the detecting of an electrochemical response also includes detecting an electrochemical response of the plurality of enzymatic reagent covered electrodes.

9. The method of claim 7 or claim 8, wherein the soluble electrochemically-active coating contains at least one of an enzymatic mediator and a redox agent.

10. The method of any one of claims 7 to 9, wherein the electrochemical response of at least one bare electrode is a current response.

11. The method of claim 10, wherein the blood sample is a whole blood sample and the current response of the at least one bare electrode is dependent on hematocrit of the whole blood sample.

12. The the method of any one of claims 7 to 10, wherein an electrochemical response of the at least one bare electrode is independent of analyte concentration of the blood sample.

13. The method of any one of claims 7 to 12, wherein the blood sample is a whole blood sample and/or wherein the analyte is glucose.

14. The method of any one of claims 7 to 13, wherein the soluble electrochemically-active coating and the at least one bare electrodes of the patterned electrically conductive layer are separated by a vertical distance in the range of 50 micrometers to 150 micrometers in the sample-receiving chamber.

**Patentansprüche**

1. Elektrochemisch basierter analytischer Teststreifen für die Bestimmung eines Analyten in einer Blutprobe, wobei der elektrochemisch basierte analytische Teststreifen umfasst:

eine elektrisch isolierende Basisschicht (110);
eine strukturierte elektrisch leitfähige Schicht (120), die auf der elektrisch isolierenden Basisschicht angeordnet ist und eine Vielzahl von Elektroden umfasst;
eine enzymatische Reagenzschicht (140a, 140b), die auf einem Teil der strukturierten leitfähigen Schicht angeordnet ist, um mindestens eine blanke Elektrode (122, 124) und eine Vielzahl von mit dem enzymatischen Reagenz bedeckten Elektroden (126, 127, 128) aus der Vielzahl von Elektroden zu definieren;
eine strukturierte Abstandsschicht (150);
eine obere Schicht (170), die eine Unterseitenfläche (176) hat; und eine lösliche elektrochemisch aktive Beschichtung (160) auf der Unterseitenfläche der oberen Schicht;
wobei zumindest die strukturierte Abstandsschicht und die obere Schicht eine probenaufnehmende Kammer (180) innerhalb des elektrochemisch basierten analytischen Teststreifens definieren;
wobei die lösliche elektrochemisch aktive Beschichtung auf der Unterseitenfläche der oberen Schicht innerhalb von mindestens einem Teil der probenaufnehmenden Kammer angeordnet ist und in einer gegenüberliegenden Beziehung zu der mindestens einen blanken Elektrode und nicht in einer gegenüberliegenden Beziehung zu der Vielzahl von reagenzbedeckten Elektroden steht;
und
wobei die lösliche elektrochemisch aktive Beschichtung enzymfrei ist.

2. Elektrochemisch basierter analytischer Teststreifen nach Anspruch 1, wobei die lösliche elektrochemisch aktive Beschichtung gegenüber der mindestens einen blanken Elektrode angeordnet ist und mit einem Abstand von der Vielzahl von enzymatischen reagenzbedeckten Elektroden, vorzugsweise mit einem Abstand im Bereich von 150 $\mu$m bis 450 $\mu$m.

3. Elektrochemisch basierter analytischer Teststreifen nach Anspruch 1 oder Anspruch 2, wobei die mindestens eine blanke Elektrode dafür ausgelegt ist, eine Stromreaktion bei Einführung einer Blutprobe in die probenaufnehmende Kammer zu erzeugen, die durch ein zugehöriges Prüftestgerät messbar ist.

4. Elektrochemisch basierter analytischer Teststreifen nach einem der Ansprüche 1 bis 3, wobei zumindest die obere Schicht und die lösliche elektrochemisch aktive Beschichtung als konstruiertes oberes Band integriert sind.

5. Teststreifen nach einem der Ansprüche 1 bis 4, wobei die lösliche elektrochemisch aktive Beschichtung mindestens ein Element aus einem enzymatischen Mediator und einem Redoxmittel enthält.

6. Teststreifen nach einem der Ansprüche 1 bis 4, wobei die lösliche elektrochemisch aktive Beschichtung und die mindestens eine blanke Elektrode von der strukturierten elektrisch leitfähigen Schicht durch einen vertikalen Abstand im Bereich von 50 $\mu$m bis 150 $\mu$m in der probenaufnehmenden Kammer getrennt sind.

7. Verfahren zum Einsatz eines analytischen Teststreifens, wobei das Verfahren umfasst: Einführen einer Blutprobe in die probenaufnehmende Kammer des elektrochemisch basierten analytischen Teststreifens nach einem der Ansprüche 1 bis 6;
wobei die Einführung derart erfolgt, dass sich die lösliche elektrochemisch aktive Beschichtung betriebsmäßig in der Blutprobe auflöst;
Feststellen einer elektrochemischen Reaktion der mindestens einen blanken Elektrode des elektrochemisch basierten analytischen Teststreifens; und
Bestimmen eines Analyten in der Blutprobe, das teilweise auf der festgestellten elektrochemischen Reaktion der mindestens einen blanken Elektrode beruht.

8. Verfahren nach Anspruch 7, wobei das Feststellen einer elektrochemischen Reaktion auch das Feststellen einer elektrochemischen Reaktion der Vielzahl von mit dem enzymatischen Reagenz bedeckten Elektroden umfasst.

9. Verfahren nach Anspruch 7 oder Anspruch 8, wobei die lösliche elektrochemisch aktive Beschichtung mindestens ein Element aus einem enzymatischen Mediator und einem Redoxmittel enthält.

10. Verfahren nach einem der Ansprüche 7 bis 9, wobei die elektrochemische Reaktion der mindestens einen blanken Elektrode eine Stromreaktion ist.

11. Verfahren nach Anspruch 10, wobei die Blutprobe eine Vollblutprobe ist, und die Stromreaktionen der mindestens einen blanken Elektrode hängt vom Hämatokrit der Vollblutprobe ab.

12. Verfahren nach einem der Ansprüche 7-10, wobei eine elektrochemische Reaktion der mindestens einen blanken Elektrode unabhängig von der Analytenkonzentration der Blutprobe ist.

13. Verfahren nach einem der Ansprüche 7 bis 12, wobei die Blutprobe eine Vollblutprobe ist, und/oder wobei der Analyt Glukose ist.

14. Verfahren nach einem der Ansprüche 7 bis 13, wobei die lösliche elektrochemisch aktive Beschichtung und die mindestens eine blanke Elektrode der strukturierten elektrisch leitfähigen Schicht durch einen vertikalen Abstand im Bereich von 50 $\mu$m bis 150 $\mu$m in der probenaufnehmenden Kammer getrennt sind.

**Revendications**

1. Bande de test analytique à base électrochimique pour la détermination d'une substance à analyser dans un échantillon de sang, la bande de test analytique à base électrochimique comprenant :

une couche de base électriquement isolante (110) ;
une couche électroconductrice à motifs (120) disposée sur la couche de base électriquement isolante et comprenant une pluralité d'électrodes ;
une couche de réactif enzymatique (140a, 140b) disposée sur une partie de la couche conductrice à motifs pour définir au moins une électrode nue (122, 124) et une pluralité d'électrodes recouvertes de réactif enzymatique (126, 127, 128) de la pluralité d'électrodes ;

une couche d'espacement à motifs (150) ;
une couche supérieure (170) ayant une surface inférieure (176) ; et
un revêtement soluble électrochimiquement actif (160) sur la surface inférieure de la couche supérieure ;
dans lequel au moins la couche d'espacement à motifs et la couche supérieure définissent une chambre de réception d'échantillon (180) au sein de la bande de test analytique à base électrochimique ;
dans lequel le revêtement soluble électrochimiquement actif est disposé sur la surface inférieure de la couche supérieure dans au moins une partie de la chambre de réception d'échantillon et dans une relation opposée à la ou aux électrodes nues et une relation non opposée à la pluralité d'électrodes recouvertes de réactif ; et
dans lequel le revêtement soluble électrochimiquement actif est exempt d'enzymes.

2. Bande de test analytique à base électrochimique selon la revendication 1, dans lequel le revêtement soluble électrochimiquement actif est disposé à l'opposé de la ou des électrodes nues et espacé de la pluralité d'électrodes recouvertes de réactif enzymatique, de préférence espacées d'une distance comprise dans l'intervalle de 150 micromètres à 450 micromètres.

3. Bande de test analytique à base électrochimique selon la revendication 1 ou la revendication 2, dans lequel la ou les électrodes nues sont configurées pour générer une réponse en courant lors de l'introduction d'un échantillon de sang dans la chambre de réception d'échantillon qui est mesurable par un appareil de mesure associé.

4. Bande de test analytique à base électrochimique selon l'une quelconque des revendications 1 à 3, dans lequel au moins la couche supérieure et le revêtement soluble électrochimiquement actif sont intégrés en une bande technique supérieure.

5. Bande de test selon l'une quelconque des revendications 1 à 4, dans lequel le revêtement soluble électrochimiquement actif contient au moins l'un parmi un médiateur enzymatique et un agent de réduction-oxydation.

6. Bande de test selon l'une quelconque des revendications 1 à 4, dans lequel le revêtement soluble électrochimiquement actif et la ou les électrodes nues de la couche électroconductrice à motifs sont séparés d'une distance verticale dans l'intervalle entre 50 micromètres à 150 micromètres dans la chambre de réception d'échantillon.

7. Procédé d'utilisation d'une bande de test analytique, le procédé comprenant :

l'introduction d'un échantillon de sang dans la chambre de réception d'échantillon de la bande de test analytique à base électrochimique selon l'une quelconque des revendications 1 à 6 ;
dans lequel l'introduction est telle que le revêtement soluble électrochimiquement actif se dissout au cours de l'opération dans l'échantillon de sang ;
la détection d'une réponse électrochimique de la ou des électrodes nues de la bande de test analytique à base électrochimique ; et
la détermination d'une substance à analyser dans l'échantillon de sang en partie sur la base de la réponse électrochimique détectée de la part de la ou des électrodes nues.

8. Procédé selon la revendication 7, dans lequel la détection d'une réponse électrochimique comprend également la détection d'une réponse électrochimique de la pluralité d'électrodes recouvertes de réactif enzymatique.

9. Procédé selon la revendication 7 ou la revendication 8, dans lequel le revêtement soluble électrochimiquement actif contient au moins l'un parmi un médiateur enzymatique et un agent de réduction-oxydation.

10. Procédé selon l'une quelconque des revendications 7 à 9, dans lequel la réponse électrochimique d'au moins une électrode nue est une réponse en courant.

11. Procédé selon la revendication 10, dans lequel l'échantillon de sang est un échantillon de sang total et la réponse en courant de la ou des électrodes nues dépend de l'hématocrite de l'échantillon de sang total.

12. Procédé selon l'une quelconque des revendications 7 à 10, dans lequel une réponse électrochimique de la ou des électrodes nues est indépendante de la concentration de la substance à analyser dans l'échantillon de sang.

13. Procédé selon l'une quelconque des revendications 7 à 12, dans lequel l'échantillon de sang est un échantillon de sang total et/ou dans lequel la substance à analyser est le glucose.

14. Procédé selon l'une quelconque des revendications 7 à 13, dans lequel le revêtement électrochimiquement actif soluble et la ou les électrodes nues de la couche électroconductrice à motifs sont séparés d'une distance verticale comprise dans l'intervalle entre 50 micromètres à 150 micromètres dans la chambre de réception d'échantillon.

**FIG. 1**

*100*

*170*

*120*

*110*

**FIG. 2**

**FIG. 3**

FIG. 4

**FIG. 5**

200

```
┌──────────────────────────────────────┐
│      INTRODUCING A BODILY FLUID SAMPLE │
│    INTO A SAMPLE-RECEIVING CHAMBER OF  │
│  AN ELECTROCHEMICAL-BASED ANALYTICAL   │
│    TEST STRIP THAT INCLUDES A SOLUBLE  │── 210
│    ELECTROCHEMICALLY-ACTIVE COATING    │
│        IN AN OPPOSING RELATIONSHIP TO  │
│             A BARE ELECTRODE           │
└──────────────────────────────────────┘
```

```
┌──────────────────────────────────────┐
│        DETECTING AN ELECTROCHEMICAL    │
│     RESPONSE OF THE BARE ELECTRODE     │── 220
│      USING AN ASSOCIATED TEST METER    │
└──────────────────────────────────────┘
```

```
┌──────────────────────────────────────┐
│       DETERMINING AN ANALYTE IN THE    │
│  BODILY-FLUID SAMPLE BASED IN PART ON THE │── 230
│   DETECTED ELECTROCHEMICAL RESPONSE    │
│          OF THE BARE ELECTRODE         │
└──────────────────────────────────────┘
```

# FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5708247 A
- US 6284125 B
- EP 0225061 A1
- EP 1742045 A1
- US 6241862 B
- US 6733655 B